# EUROPEAN PATENT APPLICATION

(11) **EP 4 272 747 A1**
(43) Date of publication of application: **08.11.2023**
(21) Application number: 22759972.7
(22) Date of filing: 17.02.2022
(51) Int. Cl.: A61K 31/7076, A61P 31/14

(54) **PHARMACEUTICAL COMPOSITION CONTAINING CARBOCYCLIC NUCLEOSIDE DERIVATIVE FOR PREVENTION AND TREATMENT OF CORONAVIRUS DISEASE 19**

(30) Priority: 26.02.2021 KR 20210026365
(71) Applicant: Future Medicine Co., Ltd., Seongnam-si, Gyeonggi-do 13449 (KR)
(72) Inventor: LEE, Hyuk Woo, Seongnam-si Gyeonggi-do 13449 (KR); SON, Seo Hyun, Seongnam-si Gyeonggi-do 13449 (KR); KANG, Ji Yoon, Seongnam-si Gyeonggi-do 13449 (KR); JANG, Hyu Jeong, Seongnam-si Gyeonggi-do 13449 (KR); SEO, Seong Wook, Seongnam-si Gyeonggi-do 13449 (KR)
(74) Representative: Roos, Peter
(86) International application number: PCT/KR2022/002329
(87) International publication number: WO 2022/182054

(57) **Abstract**

The present invention relates to a pharmaceutical composition for preventing or treating coronavirus disease 19 (COVID-19) and specifically, to a pharmaceutical composition containing a carbocyclic nucleoside derivative represented by Chemical Formula A-1 or A-2 or a pharmaceutically acceptable salt thereof.

## Description

### [Technical Field]

The present invention relates to a pharmaceutical composition for preventing and treating coronavirus disease 19, containing a carbocyclic nucleoside derivative, and specifically, to a pharmaceutical composition containing a carbocyclic nucleoside derivative that has excellent inhibit of infection and low cytotoxicity against SARS-CoV-2, and thus can be used to prevent or treat coronavirus disease 19.

### [Background Art]

Viruses are one of the leading causes of numerous intractable diseases that threaten human health, and a huge amount of capital is being invested worldwide to prevent or treat viruses. Recently, coronavirus disease 19 (COVID-19), a respiratory infectious disease caused by a new type of severe acute respiratory syndrome coronavirus (SARS-CoV-2) that first emerged in Wuhan, China, has spread all over the world, causing a pandemic. Therefore, although there is an urgent need to develop a therapeutic agent for the viruses, which can suppress their proliferation, there is currently no therapeutic or preventive agent capable of effectively and safely suppressing these viruses.

Meanwhile, antiviral agents can suppress viruses through various mechanisms, and it is known that the following two mechanisms among them can be used to effectively suppress viruses.

The first is to suppress S-adenosylhomocysteine (hereinafter referred to as SAH) hydrolase. SAH hydrolase is an enzyme in the form of a tetramer, which uses NAD⁺ as a coenzyme, and is an enzyme which plays a role in reversibly hydrolyzing SAH to adenosine (Ado) and homocysteine (Hcy) and is very important for the methylation of not only proteins, lipids and nucleic acids *in vivo,* but also materials in the body, such as histamine and norepinephrine.

The suppression of the SAH hydrolase induces the accumulation of SAH, and excess SAH sequentially suppresses S-adenosylmethionine(AdoMet)-dependent transmethylase and capping of viral mRNA to prevent proteins necessary for viral replication from being properly produced, thereby exhibiting an antiviral effect.

Since most animal DNA viruses as well as RNA viruses essentially require methyltransferases (viral mRNA guanosine N7-methytransferases, O-2'-methytransferase) for mRNA capping, the SAH hydrolase is considered an essential element in the development of a wide range of antiviral agents. That is, the development of therapeutic agents for RNA viruses and the development of SAH hydrolase inhibitors are considered to have a high correlation.

The second is to inhibit a viral RNA polymerase. RNA viruses are replicated by inserting nucleoside-5'-triphosphate (NTP), which is a substrate, into the RNA chain by RNA polymerase. Therefore, materials that inhibit RNA polymerase can also serve as antiviral agents, and when materials, which are converted into triphosphates in the body to selectively suppress viral RNA polymerase, or directly inserted into viral RNA chains to induce a chain termination reaction, are used, it is believed that effective antiviral agents can be developed.
Tetrahedron: Asymmetry, 2002, 13, 1189-1193
J. Med. Chem. 2001, 44, 3985-3993

### [Disclosure]

### [Technical Problem]

Thus, a problem to be solved by the present invention is to provide a pharmaceutical composition for preventing or treating coronavirus disease 19 (COVID-19) that is causing a serious danger worldwide by discovering a carbocyclic nucleoside derivative that has excellent ability to inhibit infection and low cytotoxicity against SARS-CoV-2, which is a type of coronavirus,

The problems of the present invention are not limited to the aforementioned technical problems, and other technical problems, which have not been mentioned, may be clearly understood by a person with ordinary skill in the art from the following description.

### [Technical Solution]

A pharmaceutical composition for preventing and/or treating coronavirus disease 19 (COVID-19) according to an exemplary embodiment of the present invention for solving the problem includes a carbocyclic nucleoside derivative represented by the following Chemical Formula A-1 or A-2 or a pharmaceutically acceptable salt thereof.

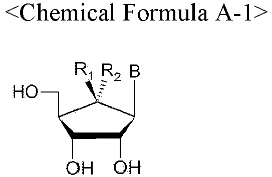

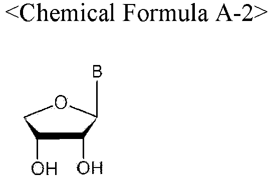

In Chemical Formula A-1 or A-2, R₁ and R₂ are each independently hydrogen or fluorine, provided that at least one of R₁ and R₂ is fluorine, and B is the following Chemical Formula B-1.

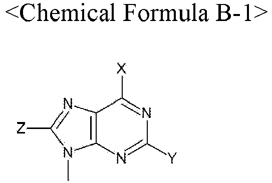

In Chemical Formula B-1, X is an amino group, an alkylamino group or an arylamino group, Y is hydrogen, a thiophenyl group or a furanyl group, and Z is hydrogen, an alkynyl group, an alkylalkynyl group or an arylalkynyl group.

The alkylamino group is -NHMe, -NHEt, -NMe₂, -NMeEt or -NEt₂, the arylamino group is phenylamino, benzylamino, halogenized phenylamino or halogenized benzylamino, the alkylalkynyl group is benzylethynyl or benzylpropynyl, and the arylalkynyl group is phenylethynyl or phenylpropynyl.

The halogenized phenylamino group or halogenized benzylamino group may be, for example, fluoro-, chloro-, bromo- or iodo-phenylamino or benzylamino, but is not limited thereto.

The alkynyl group may be, for example, ethynyl, propynyl or butynyl, but is not limited thereto.

Specifically, Chemical Formula A-1 may be the following Chemical Formula 1-1 or 1-2, and Chemical Formula A-2 may be the following Chemical Formula 2.

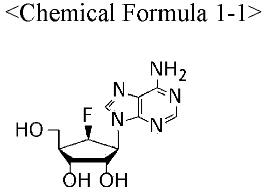

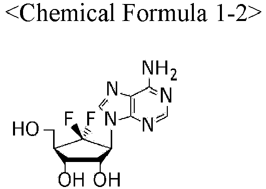

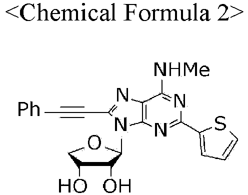

A pharmaceutical composition for preventing and/or treating COVID-19 according to an exemplary embodiment of the present invention may include the above-described carbocyclic nucleoside derivative as an active ingredient against SARS-CoV-2. That is, exemplary embodiments of the present invention may be an antiviral agent or anti-SARS-CoV-2 agent against SARS-CoV-2, containing the above-described carbocyclic nucleoside derivative.

The antiviral agent means a pharmaceutical composition that can be used not only for suppressing viral activity, replication, and the like, but also for preventing and/or treating all diseases induced by viruses.

The carbocyclic nucleoside derivative or salt may have excellent ability to inhibit infection and low cytotoxicity against SARS-CoV-2. Specifically, the carbocyclic nucleoside derivative or salt may have an IC₅₀ value of 7.5 µM or less, a CC₅₀ value of 50 or more and/or an SI value of 6.5 or more against SARS-CoV-2. More specifically, the carbocyclic nucleoside derivative or salt may have an IC₅₀ value of 4 µM or less and/or an SI value of 16 or more against SARS-CoV-2.

Alternatively, the carbocyclic nucleoside derivative or salt may have an inhibition rate of infection of 70% or more against SARS-CoV-2 at a concentration of 10 µM. At the same time, the viable cell ratio may be 70% or more. The inhibition rate of infection may be a relative rate derived compared to uninfected cells (infectivity 0%) and infected cells (infectivity 100%) not treated with the carbocyclic nucleoside derivative or salt.

The carbocyclic nucleoside derivative represented by Chemical Formula A-1 or A-2 may be provided in the form of a pharmaceutically acceptable salt. As the salt, acid addition salts formed by various organic or inorganic acids, which are pharmaceutically acceptable, are useful. As a suitable organic salt, it is possible to use, for example, carboxylic acid, phosphonic acid, sulfonic acid, acetic acid, propionic acid, octanoic acid, decanoic acid, glycolic acid, lactic acid, fumaric acid, succinic acid, adipic acid, malic acid, tartaric acid, citric acid, glutamic acid, aspartic acid, maleic acid, benzoic acid, salicylic acid, phthalic acid, phenylacetic acid, benzenesulfonic acid, 2-naphthalenesulfonic acid, methyl sulfuric acid, ethyl sulfuric acid, dodecyl sulfuric acid, and the like, and for example, halogenic acid such as hydrochloric acid and sulfuric acid, or phosphoric acid, or the like may be used.

However, the organic salt is not limited thereto, and the carbocyclic nucleoside derivative of the present invention may also be provided in the form of all salts, hydrates and solvates which may be prepared by typical methods.

The pharmaceutical composition for preventing and treating COVID-19 may be administered systemically or locally, and may be formulated using an excipient (or a diluent) such as a filler, an extender, a binder, a wetting agent, a disintegrating agent, and a surfactant, which may be generally used for oral or parenteral administration. Hereinafter, excipients and formulation methods will be specifically illustrated, but are not limited to these examples.

A solid formulation for oral administration may include a tablet, a pill, a powder, a granule, a capsule, and the like, and the solid formulation may be prepared by mixing at least one excipient, for example, starch, calcium carbonate, sucrose or lactose, gelatin, and the like with one or more compounds of Chemical Formula A-1 or A-2. Furthermore, in addition to simple excipients, lubricants such as magnesium stearate and talc may also be used. A liquid formulation for oral administration may include a suspension, a liquid for internal use, an emulsion, a syrup, and the like, and the liquid formulation may include, in addition to water and liquid paraffin which are simple diluents typically used, various excipients, for example, a wetting agent, a sweetener, a fragrance, a preservative, and the like.

A formulation for parenteral administration may include an injection, an emulsion, an inhalant, a suppository and the like. The injection may include propylene glycol, polyethylene glycol, a vegetable oil such as olive oil, and sterile aqueous and non-aqueous solvents and suspensions of esters such as ethyl oleate, and the suppository may include Witepsol, Macrogol, Tween 61, cacao butter, laurin butter, glycerogelatin and the like as a base. Further, the antiviral agent of the present invention may also be formulated as ointments or creams for topical application.

A preferred dose of the pharmaceutical composition varies depending on various factors such as the condition and body weight of a patient, the degree of a disease, the form of drug, the administration route, and the duration, but may be appropriately selected by the person skilled in the art. Further, the administration route may be changed depending on the condition of a patient and the severity thereof.

Specific details of other embodiments are included in the detailed description.

### [Advantageous Effects]

The carbocyclic nucleoside derivative according to exemplary embodiments of the present invention has excellent ability to inhibit infection and low cytotoxicity against SARS-CoV-2, and thus, may be used as a safe and effective agent for preventing and treating coronavirus disease 19 (COVID-19).

The effects according to the embodiments of the present invention are not limited to the contents exemplified above, and more various effects are included in the present specification.

### [Description of Drawings]

FIG. 1 is a flowchart showing the SARS-CoV-2 infection analysis process through HTS automation according to the Experimental Example of the present invention.
FIG. 2 is a plate map in which each well of the 384-tissue culture plate according to the Experimental Example of the present invention is classified according to treatment materials and methods.
FIG. 3 is a view showing the principle of analyzing images using the IM software according to the Experimental Example of the present invention.
FIG. 4 is an image obtained by heatmap analysis of each plate treated according to the Experimental Example of the present invention.
FIGS. 5 to 7 are graphs showing ability to inhibit infection and cytotoxicity of each well treated with compounds according to the Experimental Example of the present invention.
FIG. 8 is a set of confocal microscopy images of wells treated with 10 µM of the three compounds finally screened according to the Experimental Example of the present invention and control wells.
FIG. 9 is a graph analyzing the performance and Z'-factor of control wells according to the Experimental Example of the present invention.
FIG. 10 is a set of graphs showing a dose response curve (DRC) derived through the analysis according to the Experimental Example of the present invention.

### [Modes of the Invention]

The benefits and features of the present invention, and the methods of achieving the benefits and features will become apparent with reference to embodiments to be described below in detail. However, the present invention is not limited to the embodiments to be disclosed below and may be implemented in various other forms, and the embodiments are only provided for rendering the disclosure of the present invention complete and for fully representing the scope of the invention to a person with ordinary skill in the technical field to which the present invention pertains, and the present invention will be defined only by the scope of the claims.

The terms used in the present specification are used merely to describe embodiments, and are not intended to limit the present invention. In the present specification, the 'and/or' includes each and all combinations of one or more of the items mentioned. Further, the singular form also includes the plural forms unless specifically stated in a phrase. The terms 'comprises' and/or 'comprising' used in the specification do not exclude the presence or addition of one or more other constituent elements in addition to the referenced constituent elements. The numerical range indicated by using '-' or 'to' indicates a numerical range including values described before and after it as a lower limit and an upper limit, respectively, unless otherwise stated. 'About' or 'approximately' means a value or numerical range within 20% of the value or numerical range described thereafter.

Further, in describing the constituent elements of the examples of the present invention, terms such as first, second, A, B, (a), and (b) may be used. These terms are merely for distinguishing one constituent element from another, and the nature, turn, or order of the corresponding constituent element is not limited by the term.

Unless otherwise defined, all the terms (including technical and scientific terms) used in the present specification will be able to be used as a meaning which may be commonly understood to a person with ordinary skill in the art to which the present invention pertains. In addition, the terms defined in generally used dictionaries are not to be interpreted ideally or excessively unless clearly and specifically defined.

Moreover, in describing the examples of the present invention, when it is determined that the specific description of relevant known configurations or functions obstructs the understanding for the examples of the present invention, the detailed description thereof will be omitted.

As used herein, 'prevention' refers to suppressing the onset of a symptom or disease in an individual who does not yet have the symptom or disease, but may suffer from the symptom or disease.

As used herein, 'treatment' refers to (a) suppression of the development (exacerbation) of a symptom or disease, (b) reduction or amelioration of a symptom or disease, or (c) elimination of a symptom or disease, from an individual.

As used herein, 'individual' refers to an animal, particularly a mammal, including a human having a symptom or disease that can be 'prevented' or 'treated' by administering the composition of the present invention.

Hereinafter, embodiments of the present invention will be described in detail with reference to Preparation Examples and Experimental Examples, but it is obvious that the effects of the present invention are not limited by the following Experimental Examples.

### Preparation Examples: Preparation of carbocyclic nucleoside derivative of present invention

### Preparation Example 1: Chemical Formula 1-1

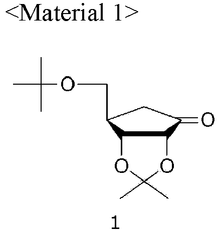

Preparation method of Material 1: Material 1 was synthesized with reference to Reference Documents [1] Tetrahedron: Asymmetry, 2002, 13, 1189-1193 and [2] J. Med. Chem. 2001, 44, 3985-3993.

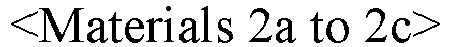

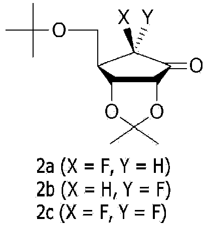

Preparation method of Materials 2a and 2b: After Material 1 (6 g, 24.8 mmol) synthesized above is dissolved in anhydrous THF, chlorotriethylsilane (TESCI) (16.7 mL, 99.2 mmol) is added dropwise thereto, and the resulting mixture is cooled to -78°C. After a 1.0 M lithium bis(trimethylsilyl)amide/THF solution (LiHMDS 1.0M solution in THF) (50 mL, 50 mmol) is slowly added dropwise to the cooled mixture, the reaction is stirred well under the same conditions for 30 minutes to perform silyl enol etherification reaction. After it is confirmed by TLC that the reaction is completed, an aqueous saturated ammonium chloride (NH₄Cl) solution is added to the reaction mixture to terminate the reaction, the aqueous solution layer is extracted with ethyl acetate, and then the organic layer is separated. The separated organic layer is sufficiently washed with water and brine, dried over magnesium sulfate (MgSO₄), and filtered under reduced pressure to remove the remaining solid, and then the residue is concentrated under reduced pressure. After the concentrated residue is dissolved in anhydrous DMF, the resulting solution is cooled to 0°C. When 1.5 equivalents of 1-chloromethyl-4-fluoro-1,4-diazoniabicyclo [2.2]octane bis(tetrafluoroborate) (trade name Selectfluor^{®}) (10.15 g, 28.65 mmol) (10.15 g, 28.65 mmol) are added dropwise to the cooled mixture, and then the resulting mixture is stirred under the same conditions for 15 hours, Materials 2a and 2b, which are fluorinated compounds, are produced at a ratio of 3:1 (confirmed by ratio in NMR). When it is confirmed by TLC that the reaction is completed, an aqueous saturated ammonium chloride (NH₄Cl) solution is added to the reaction mixture to terminate the reaction, the aqueous solution layer is extracted with ethyl acetate, and then the organic layer is separated. The separated organic layer is sufficiently washed with water and brine, dried over magnesium sulfate (MgSO₄), and filtered under reduced pressure to remove the remaining solid, and then the residue is concentrated under reduced pressure. The concentrated residue can be separated and purified by silica gel chromatography or the like to obtain 2a (6.591 g, 58%) and 2b (3.078 g, 27%), respectively.
2a: ¹H NMR (400 MHz, CDCl₃) 5.29 (dd, *J* = 8.2, 49.5 Hz, 1 H), 4.70 (t, *J* = 5.7 Hz, 1 H), 4.20 (dd, *J* = 2.4, 6.1 Hz, 1 H), 3.61 (dd, *J* = 1.6, 8.6 Hz, 1 H) 3.38-3.41 (m, 1 H), 2.75 (d, *J* = 8.2 Hz, 1 H), 1.41 (s, 3 H), 1.30 (s, 3 H), 1.06 (s, 9 H)
2b: ¹H NMR (600 MHz, CDCl₃) 5.21-5.36 (ddd, *J* = 1.3, 4.5, 50.8 Hz, 1 H,), 4.55 (d, *J* = 5.9 Hz, 1 H), 4.50 (d, *J* = 5.9 Hz, 1 H), 3.63 (d, *J* = 2.2 Hz, 2 H), 2.52-2.58 (m, 1 H), 1.41 (s, 3 H), 1.33 (s, 3 H), 1.13 (s, 9 H)

Preparation Method of Material 2c: When synthesis is performed on Material 2a (3.29 g, 12.6 mmol) synthesized above in the same manner as in the process of synthesizing Materials 2a and 2b from Material 1 specified above, 2c (2.95 g, 84%) can be synthesized.

¹H NMR (500 MHz, CDCl₃) 4.72 (t, *J* = 6.1 Hz, 1 H), 4.35-4.38 (m, 1 H), 3.68 (d, *J* = 8.6 Hz, 1 H), 3.68 (d, *J* = 8.6 Hz, 1 H) 3.46 (d, *J* = 8.5 Hz, 1 H), 2.67 (d, *J* = 17.4 Hz, 1 H), 1.42 (s, 3 H), 1.33 (s, 3 H), 1.05 (s, 9 H). (mixed with peaks presumed to be those of a material equilibrated in the form of diol)

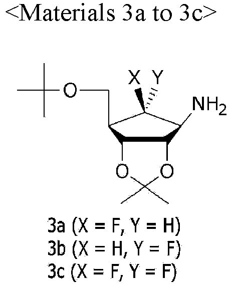

Preparation Method of Material 3a: After Material 2a (3.33 g, 12.8 mmol) is dissolved in methanol, the resulting solution is sufficiently cooled to 0°C or less. Sodium borohydride (NaBH₄) (1.45 g, 38.4 mmol) is slowly added to the cooled solution. Thereafter, the reaction is stirred under the same conditions, and after it is confirmed by TLC that the reaction is completed, an aqueous saturated ammonium chloride (NH₄Cl) solution is added to the reaction mixture to terminate the reaction, the aqueous solution layer is extracted with ethyl acetate, and then the organic layer is separated. The separated organic layer is sufficiently washed with water and brine, dried over magnesium sulfate (MgSO₄), and filtered under reduced pressure to remove the remaining solid, and then the residue is concentrated under reduced pressure. After an intermediate (2.54 g, 76%) obtained by separating and purifying the resulting alcohol compound by silica gel chromatography or the like is dissolved in anhydrous pyridine, the resulting solution is cooled to 0°C. After trifluoromethanesulfonic anhydride (3.26 ml, 19.36 mmol) was added dropwise to the cooled mixture, the resulting mixture was stirred under the same conditions for 30 minutes to perform trifluoromethyl sulfonation. After it is confirmed by TLC that the reaction is completed, an aqueous saturated ammonium chloride (NH₄Cl) solution is added to the reaction mixture to terminate the reaction, the aqueous solution layer is extracted with ethyl acetate, and then the organic layer is separated. The separated organic layer is sufficiently washed with water and brine, dried over magnesium sulfate (MgSO₄), and filtered under reduced pressure to remove the remaining solid, and then the residue is concentrated under reduced pressure. After the resulting trifluoromethyl sulfonated compound and sodium azide (NaN₃) (1.89 g, 29.04 mmol) are mixed with anhydrous DMF, the resulting mixture is stirred while being heated to 60°C to perform azidation. After stirring is performed for about 4 hours, it is confirmed by TLC that the reaction is completed, the reaction is terminated with water, and then the aqueous solution layer was extracted with ethyl acetate, and then the organic layer was separated. The separated organic layer is sufficiently washed with water and brine, dried over magnesium sulfate (MgSO₄), and filtered under reduced pressure to remove the remaining solid, and then the residue is concentrated under reduced pressure. The obtained azide compound is separated and purified by silica gel chromatography or the like, and the obtained compound (4.07 g, 42%) is dissolved in methanol. An appropriate amount of palladium/carbon is added to the corresponding solution, and the reaction vessel is charged with hydrogen to perform the hydrogenation reaction, thereby reducing an azide group to an amine group. When it is confirmed by TLC that the reaction is completed, and then the reaction is completed, the remaining solid is removed by filtration under reduced pressure, and a desired Material 3a can be obtained by concentration under reduced pressure. The obtained Material 3a proceeds to the next process without any separation and purification process.
¹H NMR (600 MHz, CDCl₃) d 4.96 (dt, *J* =52.5, 3.3 Hz, 1 H), 4.31-4.36 (m, 2 H), 3.48-3.54 (m, 2 H), 3.27 (td, *J* = 4.1, 28.4 Hz, 1 H), 2.24-2.34 (m, 1 H), 1.80 (s, 2 H), 1.45 (s, 3H), 1.26 (s, 3H), 1.16 (s, 9 H)

Preparation Method of Material 3c: When synthesis is performed on Material 2c (4.95 g, 17.79 mmoL) synthesized above in the same manner as in the process of synthesizing Material 3a from Material 2a specified above, 3c (2.98 g, 60%) can be synthesized. However, in the reaction to convert ketone into alcohol, lithium borohydride (LiBH₄) is used instead of sodium borohydride (NaBH₄), and in the azidation reaction, 10 equivalents of sodium azide should be used, the temperature should be increased to 100°C, and the stirring time should be increased to about 15 hours.

¹H NMR (800 MHz, CDCl₃) d 4.45-4.46 (m, 1 H), 4.25-4.27 (m, 1 H), 3.58 (dd, *J* = 4.5, 9.2 Hz, 1 H), 3.54 (dd, *J* = 5.1, 9.2 Hz, 1 H), 3.35-3.38 (m, 1 H), 2.56-2.59 (m, 1 H), 1.94 (bs, 3 H), 1.47 (s, 3 H), 1.28 (s, 3 H), 1.18 (s, 9 H)

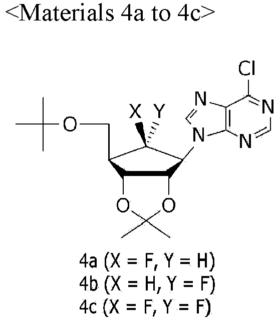

Preparation Method of Material 4a: After Material 3a (982 mg, 3.757 mmol) synthesized above, 5-amino-4,6-dichloropyrimidine (1.85 g, 11.27 mmol), and diisopropylethylamine (DIPEA) (6.54 mL, 37.57 mmol) are mixed with n-butanol, the resulting mixture is heated to 170°C and stirred for 4 hours using a microwave or the like. After it is confirmed by TLC that the reaction is completed, the reaction mixture is mixed with methanol and concentrated under reduced pressure, the residue can be separated and purified by silica gel chromatography or the like to obtain an intermediate (964 mg, 66%). After this intermediate is dissolved in diethoxymethyl acetate, the resulting solution is stirred at 140°C for about 3 hours using a microwave or the like. After it is confirmed by TLC that the reaction is completed, the reaction mixture is mixed with methanol and concentrated under reduced pressure, and then the resulting mixture can be separated and purified by silica gel chromatography or the like to obtain Material 4a (643 mg, 65%).
¹H NMR (400 MHz, CDCl₃) δ 8.74 (s, 1 H), 8.34 (d, *J* = 2.4 Hz, 1 H), 5.28-5.43 (dt, *J* = 52.8, 2.8 Hz, 1 H), 5.12-5.23 (m, 2 H), 4.61 (t, *J* = 5.0 Hz, 1 H), 3.65-3.69 (m, 1 H), 3.61 (t, *J* = 9.2 Hz, 1 H), 2.56-2.71 (m, 1 H), 1.56 (s, 3 H), 1.32 (s, 3 H), 1.17 (s, 9 H)

Preparation Method of Material 4c: When synthesis is performed on Material 3c (182 mg, 0.625 mmol) synthesized above in the same manner as in the process of synthesizing Material 4a from Material 3a specified above, 4c (223 mg, 50%) can be synthesized. However, in the first reaction step, the temperature should be increased to 200°C and the stirring time should be increased to about 7 hours.

¹H NMR (300 MHz, CDCl₃) δ 8.76 (s, 1 H), 8.76 (s, 1 H), 8.29 (d, *J* = 2.4 Hz, 1 H), 5.26-5.37 (m, 1 H), 5.11 (t, *J* = 6.9 Hz, 1 H), 4.59-4.64 (m, 1 H), 3.64-3.74 (m, 2 H), 2.81-2.96 (m, 1 H), 1.58 (s, 3 H), 1.33 (s, 3 H), 1.20 (s, 9 H)

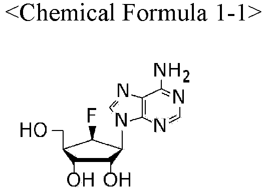

Preparation Method of Chemical Formula 1-1: After Material 4a (220 mg, 0.54 mmol) synthesized above is dissolved in tert-butanol, the resulting solution is transferred to a high-temperature sealed vessel (stainless steel bomb reactor). After saturated ammonia/tert-butanol (tertiary butanol) is added dropwise to the mixed solution, the reaction vessel is sealed and stirred at 120°C for about 15 hours. After it is confirmed by TLC that the reaction is completed, the reaction mixture is diluted with methanol and then concentrated under reduced pressure. After the concentrated residue is dissolved in THF, a solution in which trifluoroacetic acid (TFA) and water are mixed at 2:1 is added dropwise to the mixed solution. After the reaction mixture is stirred at 60°C for about 15 hours, it is confirmed by TLC that the reaction is completed, and the mixture is concentrated under reduced pressure. The concentrated residue can be separated and purified by silica gel chromatography or the like to obtain a compound of Chemical Formula 1-1 (66 mg, 43%) as a final material.
¹H NMR (400 MHz, CD₃OD) δ 8.31 (d, *J* = 2.4 Hz, 1 H), 8.23 (s, 1 H), 5.36-5.39 (m, 1 H), 5.20-5.35 (td, *J* = 2.8, 53.6 Hz, 1 H), 5.05-5.16 (m, 1 H), 4.66-4.69 (m, 1 H), 3.64-3.71 (m, 2 H), 2.51-2.66 (m, 1 H), 1.55 (s, 3 H), 1.35 (s, 3 H), 1.22 (s, 9 H)

### Preparation Example 2: Chemical Formula 1-2

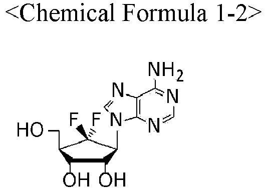

When synthesis is performed on Material 4c (223 mg, 0.534 mmol) synthesized above in the same manner as in the process of synthesizing Chemical Formula 1-1 from Material 4a specified above, a compound of Chemical Formula 1-2 (99 mg, 61%) can be synthesized.
¹H NMR (400 MHz, CD₃OD) δ 8.29 (s, 1 H), 8.21 (s, 1 H), 5.29-5.36 (m, 2 H), 4.66 (bs, 1 H), 3.75-3.79 (m, 1 H), 3.66-3.70 (m, 1 H), 2.80-2.89 (m, 1 H), 1.57 (s, 3 H), 1.34 (s, 3 H), 1.22 (s, 9 H)

### Preparation Example 3: Chemical Formula 2

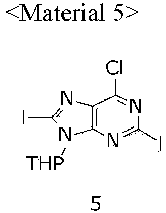

Preparation Method of Material 5: 2,2,6,6-tetramethylpiperidine (TMP, 59.2 g, 419 mmol) is dissolved in anhydrous THF (170 mL) under nitrogen gas, n-BuLi (285 mL, 2.5 M in hexane, 457 mmol) is slowly added thereto at -78°C, and the resulting mixture is stirred at 0°C for 2 hours. The reaction mixture is again cooled to -78°C, a starting material 6-chloro-9-(tetrahydro-2H-pyran-2-yl)-9H-purine (20 g, 84 mmol) dissolved in anhydrous THF (110 mL) is slowly added thereto, and the resulting mixture is stirred for 3 hours. Iodine (102 g, 402 mmol) dissolved in THF (200 mL) is slowly added to the reaction mixture at -78°C, the resulting mixture is stirred at the same temperature for 3 hours, then the temperature is gradually increased to 0°C over 5 hours. After it is confirmed that the reaction is completed, a 10% sodium thiosulfate solution (550 mL) and an aqueous saturated NH₄Cl solution (250 mL) are added thereto to terminate the reaction. The reaction mixture is extracted with EtOAC (500 mL), dried over anhydrous MgSO₄ and concentrated under reduced pressure. The remaining residue is purified by flash column chromatography under the condition of CH₂Cl₂/MeOH = 100/1 to obtain the desired target compound 5 (66%) in the form of an ivory solid.

¹H NMR (600 MHz, CDCl₃) *δ* 5.66 (dd, *J* = 2.4, 10.8 Hz, 1H), 4.20 (m, 1 H), 3.74 (td, *J*= 2.4, 12 Hz, 1H), 3.04 (m, 1 H), 2.17 (m, 1 H), 1.62-1.91 (m, 4 H).

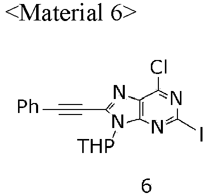

Preparation Method of Material 6: After Starting Material 5 (500 mg, 1.01 mmol) is well dissolved in anhydrous DMF (10 mL) and toluene (10 mL), bis(dibenzylideneacetone)palladium (0) (57.5 mg, 0.10 mmol), copper (I) iodide (38.4 mg, 0.20 mmol), diisopropylamine (306.60 mg, 3.03 mmol), phenylacetylene (1.1 eq.) are added thereto, and the resulting mixture is sufficiently stirred. The reaction mixture is stirred at room temperature under nitrogen gas for 12 hours. After the reaction is completed, the reaction mixture is extracted with EtOAC (20 mL), dried over anhydrous MgSO₄ and concentrated. The residue is purified by column chromatography under the condition of hexanes/EtOAc = 4/1 to obtain the target compound 6 (75%).

¹H NMR (600 MHz, CDCl₃) δ 7.66-7.64 (m, 2H), 7.52-7.42 (m, 3H), 5.88 (dd, *J* = 11.3, 2.1 Hz, 1H), 4.23 (d, *J* = 11.5 Hz, 1H), 3.80-3.74 (m, 1H), 2.92 (qd, *J* = 12.1, 4.0 Hz, 1H), 2.17-2.14 (m, 1H), 1.96-1.55 (m, 4H).

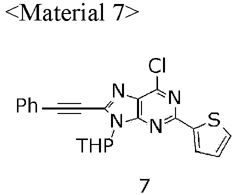

Preparation Method of Material 7: Starting Material 6 is dissolved in anhydrous THF (0.13 M), 0.1 equivalents of bis(triphenylphosphine)palladium(II) dichloride and 2 equivalents of 2-tributylstannylthiophene are added thereto, and the resulting mixture is stirred at room temperature under nitrogen gas substitution conditions. The above mixture is heated and stirred at 60°C for 3 hours, then cooled to room temperature and evaporated. The remaining material is separated by column chromatography (silica gel, hexanes/EtOAC, 15/1) to obtain the final product 7.

¹H NMR (600 MHz, DMSO) δ (ppm): 7.90 (dd, 1 H, *J* = 8.0, 6.8 Hz), 7.43 (m, 2 H), 7.46 (m, 3 H), 7.42 (dd, 1 H, *J* = 3.6, 0.8 Hz), 7.11 (dd, 1 H, *J* = 3.6, 1.6 Hz), 5.79 (dd, 1 H, *J*= 11.2, 2.4 Hz), 4.09 (d, 1 H, *J* = 11.2 Hz), 3.70 (m, 1 H), 3.50 (m, 1H), 2.69 (t, 2 H, *J* = 6.8 Hz), 2.03 (m, 2 H), 1.74 (m, 1 H).

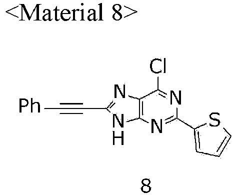

Preparation Method of Material 8: After anhydrous ethanol (0.13 M) is added to the starting material, 7, the resulting mixture is stirred, 0.2 equivalents of pyridinium p-toluenesulfonate are added thereto at room temperature under nitrogen gas substitution. After the mixture is stirred at the same temperature for 4 hours, triethylamine (1 mL) is added to terminate the reaction, and then the resulting mixture is evaporated. The reaction product is separated by column chromatography to obtain the final product 8 (60%).

¹H NMR (600 MHz, CDCl₃) δ 8.06 (d, *J* = 3.2 Hz, 1H), 7.51-7.35 (m, 6H), 7.13 (t, *J* = 4.3 Hz, 1H).

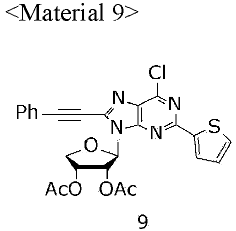

Preparation Method of Material 9: After Starting Material 8 (0.5 mmol) is sufficiently dissolved in EDC (5 mL), N,O-bis(trimethylsilyl)acetamide (BSA) (1.44 eq.) is slowly added dropwise thereto at room temperature. After the reaction mixture is stirred at 40°C for 1 hour, sugar 3 (1.2 eq.) dissolved in EDC (2 mL) is slowly added dropwise thereto at room temperature. After the reaction mixture is cooled to 0°C, TMSOTf (0.68 eq.) is added thereto, and the resulting mixture is warmed to 80°C and stirred for 3 hours. When the reaction is completed, the reaction mixture is treated with an aqueous saturated NaHCO₃ solution and extracted with CH₂Cl₂. The residue is concentrated and purified by column chromatography to obtain the target compound 9 (58%).

¹H NMR (600 MHz, CDCl₃) δ 8.04 (d, *J* = 3.2 Hz, 1H), 7.66 (d, *J* = 6.9 Hz, 2H), 7.51-7.42 (m, 4H), 7.15 (t, *J* = 4.3 Hz, 1H), 6.41 (s, 2H), 5.95 (s, 1H), 4.93 (dd, *J* = 10.5, 4.1 Hz, 1H), 4.27 (d, *J* = 10.5 Hz, 1H), 2.22 (s, 3H), 2.08 (s, 3H).

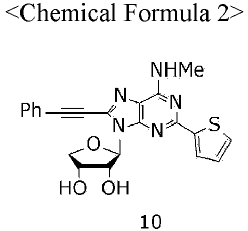

Preparation Method of Chemical Formula 2: After anhydrous ethanol (0.3 M) is added to Starting Material 9 and the resulting mixture is stirred, trimethylamine (4 eq.) and methylamine hydrochloride (1.3 eq.) were added thereto, and the resulting mixture is stirred at room temperature under nitrogen gas filling. After the mixture is stirred at the same temperature for 24 hours and evaporated, the resulting mixture is separated by column chromatography to obtain the final material Chemical Formula 2 (Material 10).

¹H NMR (600 MHz, MeOD) *δ* 7.87 (dd, *J* = 1.0, 3.6 Hz, 1 H), 7.63-7.67 (m, 2 H), 7.41-7.50 (m, 4 H), 7.08 (dd, *J* = 3.6, 5.0 Hz, 1 H), 6.22 (d, *J* = 6.0 Hz, 1 H), 5.46 (dd, *J* = 4.7, 5.8 Hz, 1 H), 4.72 (dd, *J* = 3.3, 9.5 Hz, 1 H), 4.05 (dd, *J* = 1.3, 9.5 Hz, 1 H), 3.17 (s, 3 H).

### Experimental Example: Test of activity against SARS-CoV-2

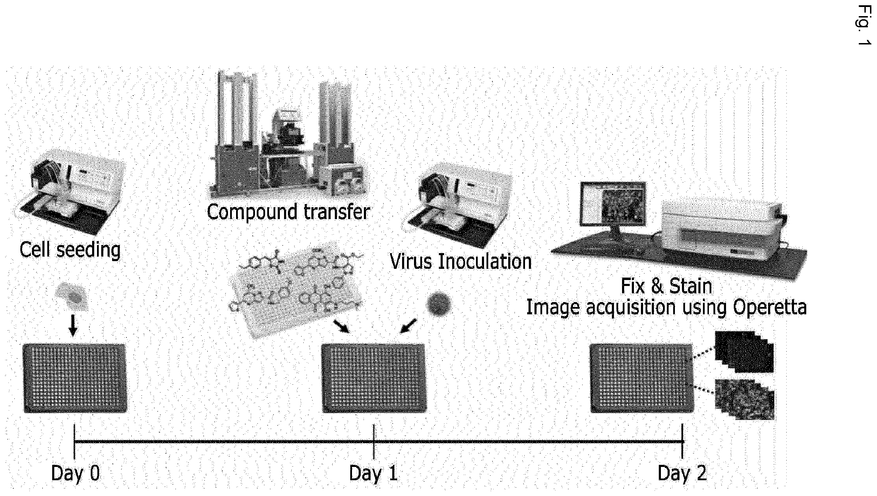

To verify the activity of the compounds of the present invention against SARS-CoV-2, the compounds were tested in a coronavirus evaluation model. For this purpose, confocal microscopy images of viral N proteins and cell nuclei were analyzed using Image Mining (IM) software, and a dose response curve (DRC) for each compound was produced. FIG. 1 is a flowchart showing the SARS-CoV-2 infection analysis process through HTS automation according to such an experimental example, and the specific experimental method is as follows.

SARS-CoV-2 was provided by the Korea Centers for Disease Control and Prevention (KCDC), and Vero cells were obtained from ATCC.

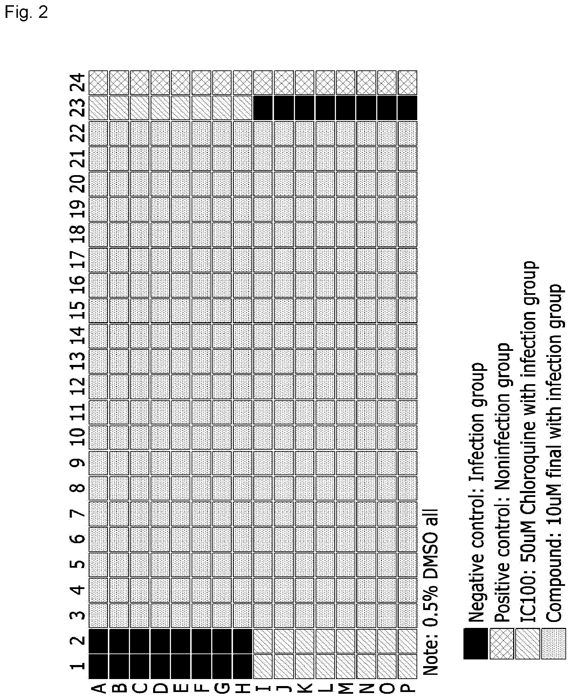

A 384-tissue culture plate was inoculated with 1.2 × 10⁴ Vero cells per well. Twenty-four hours later, the cells were treated with compounds prepared in two-fold serial dilutions in DMSO at 10 points, with the highest concentration of 50 µM. FIG. 2 is a plate map in which each well of the 384-tissue culture plate is classified according to treatment materials and methods. In FIG. 2, blue 'Compound' wells indicates wells treated with 84 compounds including the compounds of the present invention. The process was performed identically on two plates (Replicates 1 and 2) for reliability verification.

About 1 hour after the compound treatment, the cells were infected with SARS-CoV-2 at MOI of 0.0125 in a BSL3 facility and cultured at 37°C for 24 hours. Thereafter, the cells were fixed with 4% paraformaldehyde (PFA), and then permeabilization was performed. Thereafter, the cells were stained by being treated with an anti-SARS-CoV-2 nucleocapsid (N) primary antibody and treated with a 488-conjugated goat anti-rabbit IgG secondary antibody and Hoechst 33342. Fluorescence expression was imaged using Operetta (Perkin Elmer), which is a large-capacity image analysis device.

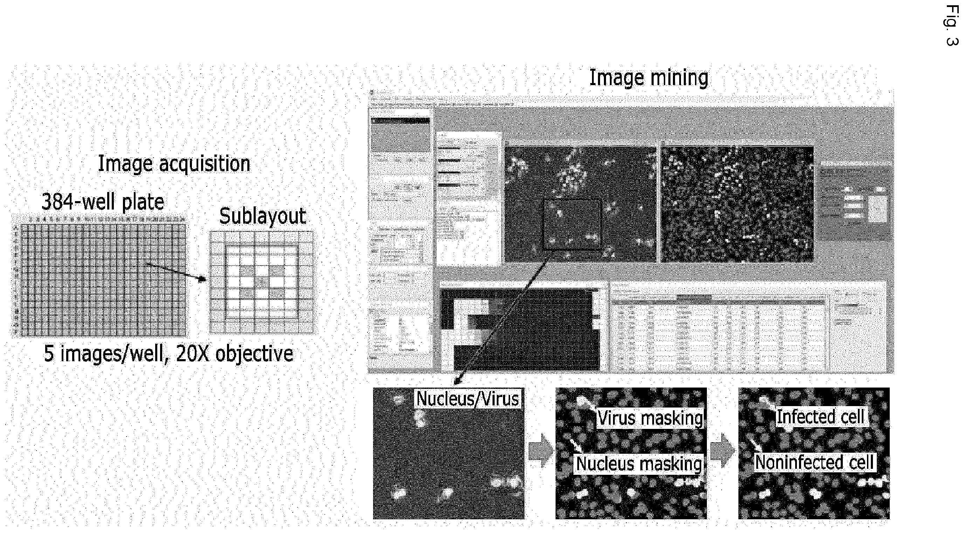

The acquired images were analyzed using Image Mining (IM) software, which is an in-house analysis program. FIG. 3 is a view showing the principle of analyzing images using the IM software.

The total number of cells per well was calculated as the number of nuclei stained with Hoechst, and the number of infected cells was calculated as the number of cells expressing nucleocapsid protein. An infection ratio was calculated as the number of cells expressing nucleocapsid protein/the total number of cells. The infection ratio per well was normalized by setting the average infection ratio of wells including uninfected cells (mock) in the same plate as 0% and the average infection of wells including infected cells not treated with the compound (0.5% DMSO group) as 100%. The response curve according to the drug concentration and IC₅₀ and CC₅₀ values were derived using the Y = Bottom + (Top Bottom)/(1 + (IC₅₀/X)Hillslope) equation running the XLFit 4 (IDBS) software. All the IC₅₀ and CC₅₀ values were determined in duplicate, and the reliability of the assays was verified by the values of Z'-factor and % coefficient of variation (%CV).

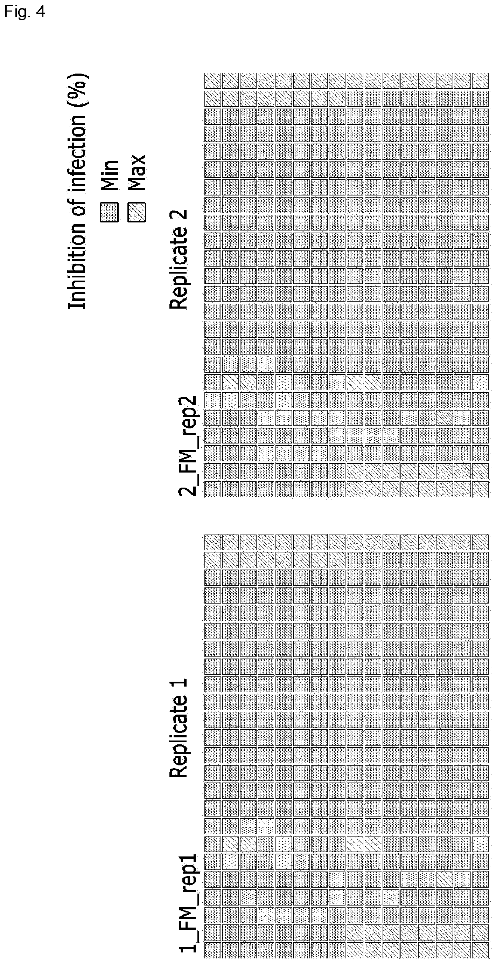

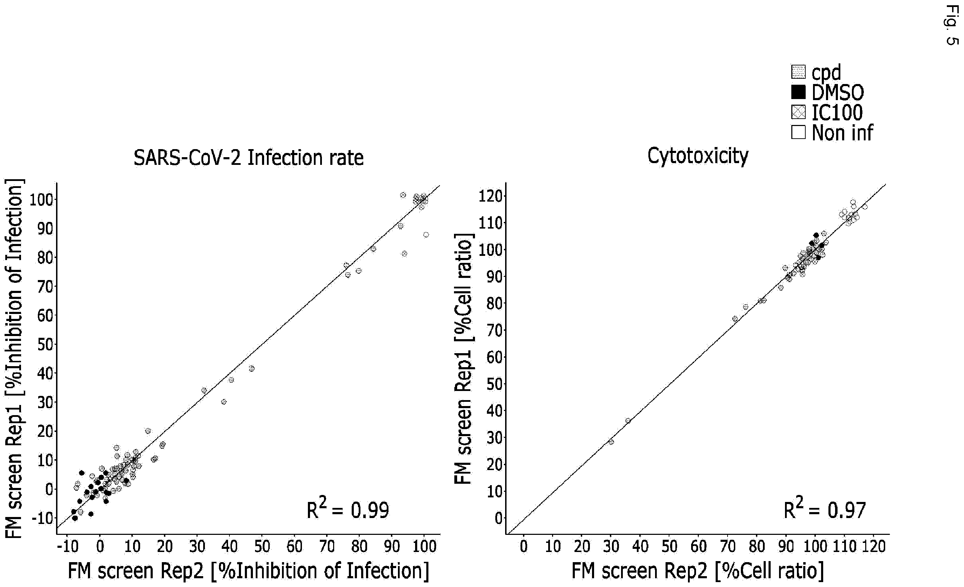

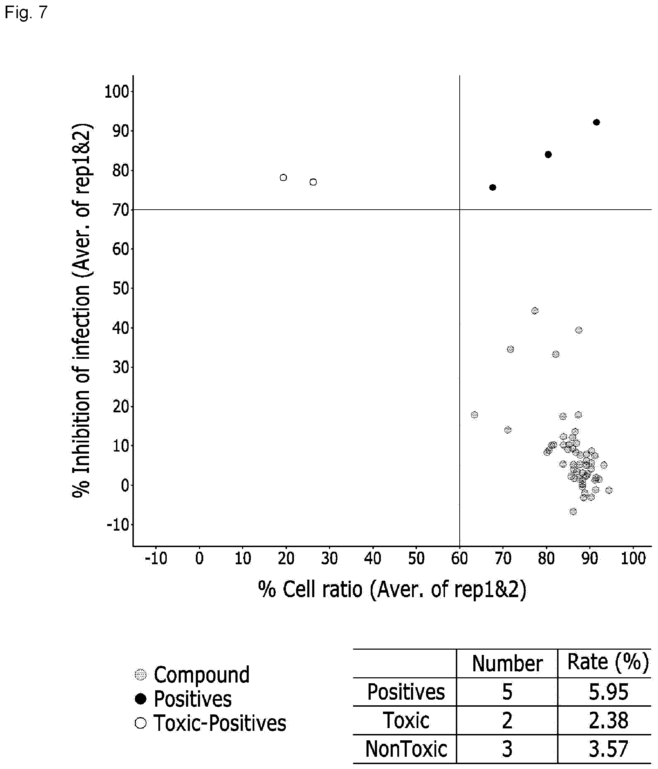

FIG. 4 is an image obtained by heatmap analysis of each plate treated according to the Experimental Example. FIGS. 5 to 7 are graphs showing ability to inhibit infection and cytotoxicity of each well treated with compounds.

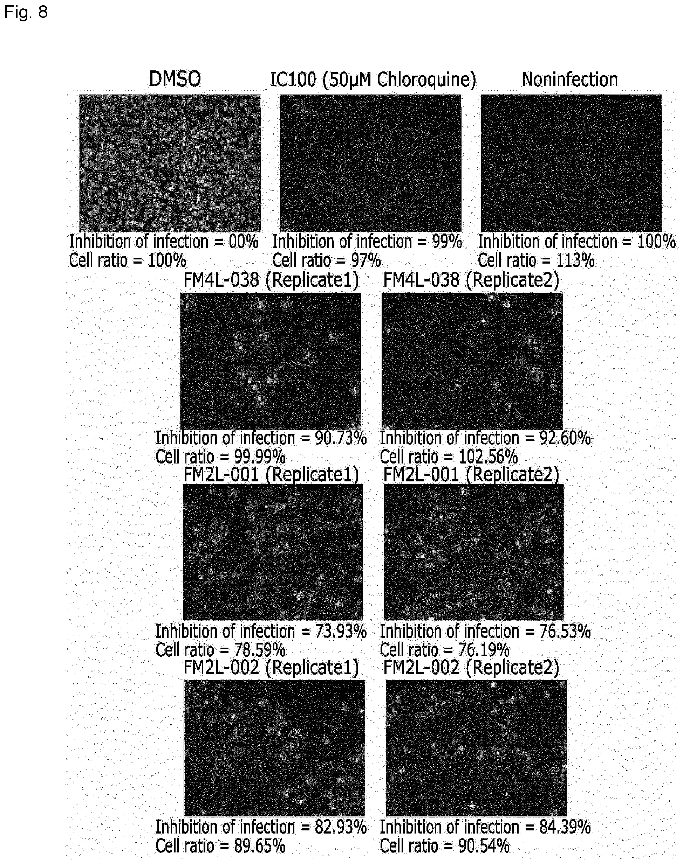

Through the image analysis results in FIG. 4 and the graphs in FIGS. 5 to 7, 5 compounds (Positives + Toxic-Positives) with an ability to inhibit infection of 70% or more were screened, and among them, 3 compounds (Positives) exhibiting cytotoxicity with a viable cell ratio of 70% or more were screened FIG. 8 is a set of confocal microscopy images of wells treated with 10 µM of the three compounds finally screened (FM4L-038, FM2L-001, FM2L-002) and control wells. FM2L-001, FM2L-002 and FM4L-038 indicate compounds of Chemical Formulae 1-1, 1-2 and 2, respectively.

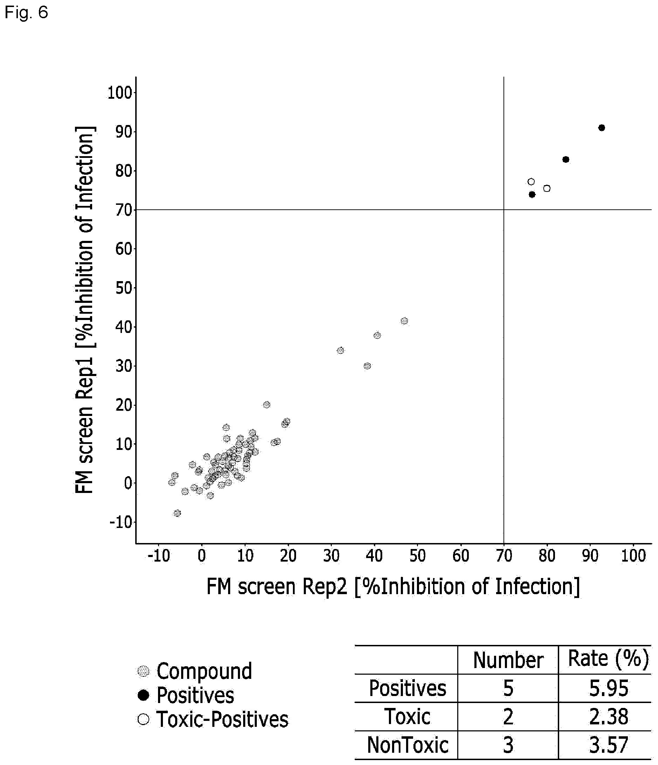

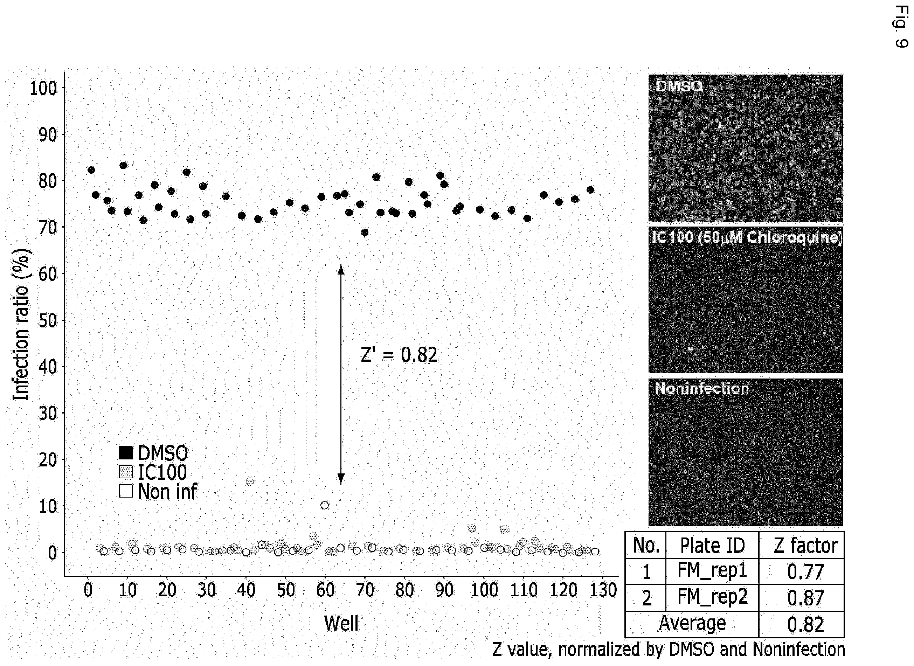

Meanwhile, the graphs showing the correlation between the two plates in FIGS. 5 and 6 support that the experiments were performed reliably. FIG. 9 is a graph analyzing the performance and Z'-factor of control wells, and the Z'-factor is obtained from the mean value and standard deviation value of the positive control and the negative control, and when it corresponds to 0.5 or more, it can be determined that the reliability of the experiment has been established Since the Z' factor of the experiment was shown to be 0.82, it can be seen that the experiment has been well verified.

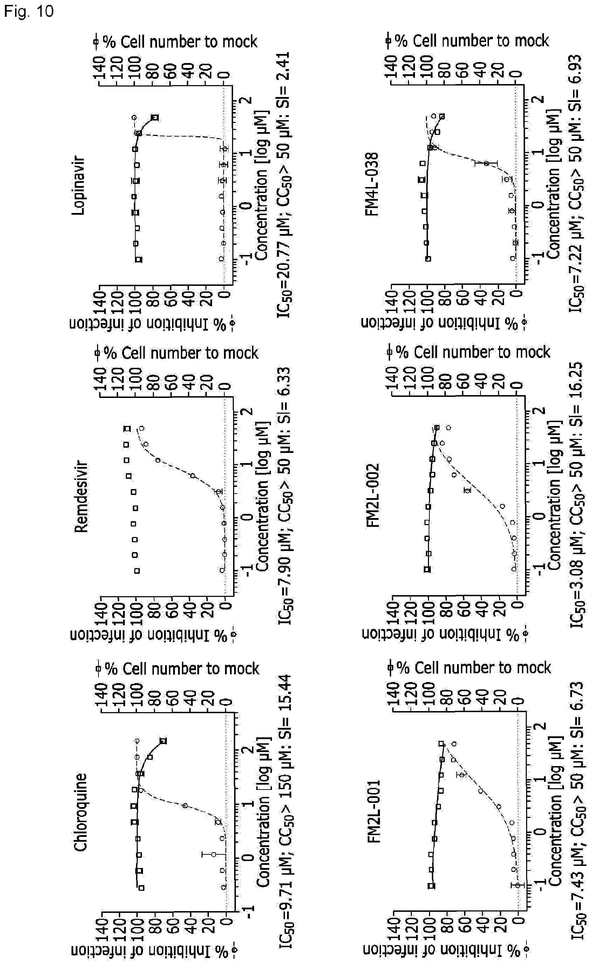

FIG. 10 is a set of graphs showing a dose response curve (DRC) derived through the analysis, and the following Table 1 shows the IC₅₀, CC₅₀ and SI values derived through the DRC for each compound. As shown in FIG. 10, the finally screened compounds of the present invention exhibit much lower IC₅₀ values than existing compounds chloroquine, remdesivir and lopinavir, which are known to be efficacious against SARS-CoV-2, and show excellent ability to inhibit infection, simultaneously exhibit similar levels of CC₅₀ values, and also have relatively high Si values, so that the compounds can be utilized as safer and more effective agent for preventing and treating an infectious disease of SARS-CoV-2 (COVID-19).

**[Table 1]**

| | IC₅₀ (µM) | CC₅₀ (µM) | **SI** |
|---|---|---|---|
| **Chemical Formula 1-1** | 7.43 | > 50 | 6.73 |
| **Chemical Formula 1-2** | 3.08 | > 50 | 16.25 |
| **Chemical Formula 2** | 7.22 | > 50 | 6.93 |
| **Chloroquine** | 9.71 | > 150 | 15.44 |
| **Remdesivir** | 7.90 | > 50 | 6.33 |
| **Lopinavir** | 20.77 | > 50 | 2.41 |

As described above, although the present invention is mainly described with reference to the embodiments of the present invention, this is merely an example and does not limit the present invention, and it will be appreciated that a person with ordinary skill in the art to which the present invention pertains can make various modifications and applications which are not exemplified above within a range not departing from the essential characteristics of the embodiments of the present invention. For example, each constituent element specifically shown in the embodiments of the present invention can be modified and implemented. And differences related to these modifications and applications should be construed as being included in the scope of the present invention defined in the appended claims.

## Claims

1. A pharmaceutical composition for preventing or treating coronavirus disease 19 (COVID-19), comprising a carbocyclic nucleoside derivative represented by the following Chemical Formula A-1 or a pharmaceutically acceptable salt thereof. 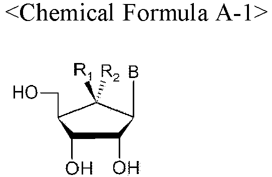 (in Chemical Formula A-1, R₁ and R₂ are each independently hydrogen or fluorine, provided that at least one of R₁ and R₂ is fluorine, and B is the following Chemical Formula B-1) 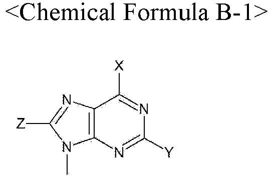 (in Chemical Formula B-1, X is an amino group, an alkylamino group or an arylamino group, Y is hydrogen, a thiophenyl group or a furanyl group, and Z is hydrogen, an alkynyl group, an alkylalkynyl group or an arylalkynyl group)

2. The pharmaceutical composition of claim 1, wherein Chemical Formula A-1 is the following Chemical Formula 1-1.

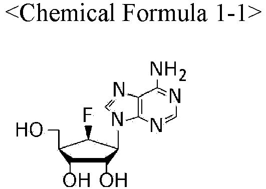

3. The pharmaceutical composition of claim 1, wherein Chemical Formula A-1 is the following Chemical Formula 1-2.

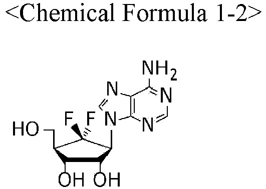

4. The pharmaceutical composition of claim 1, wherein the carbocyclic nucleoside derivative or salt has an IC₅₀ value of 7.5 µM or less, a CC₅₀ value of 50 or more and an SI value of 6.5 or more against SARS-CoV-2.

5. The pharmaceutical composition of claim 4, wherein the carbocyclic nucleoside derivative or salt has an IC₅₀ value of 4 µM or less and an SI value of 16 or more against SARS-CoV-2.

6. A pharmaceutical composition for preventing or treating coronavirus disease 19 (COVID-19), comprising a carbocyclic nucleoside derivative represented by the following Chemical Formula A-2 or a pharmaceutically acceptable salt thereof. 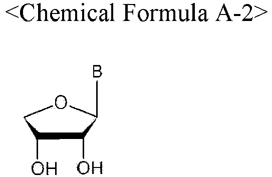 (in Chemical Formula A-2, B is the following Chemical Formula B-1) 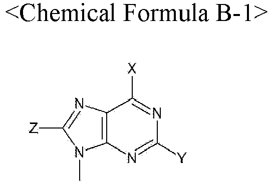 (in Chemical Formula B-1, X is an amino group, an alkylamino group or an arylamino group, Y is hydrogen, a thiophenyl group or a furanyl group, and Z is hydrogen, an alkynyl group, an alkylalkynyl group or an arylalkynyl group)

7. The pharmaceutical composition of claim 6, wherein in Chemical Formula B-1, X is an alkylamino group or an arylamino group, Y is a thiophenyl group or a furanyl group, and Z is an alkynyl group, an alkylalkynyl group or an arylalkynyl group, provided that
the alkylamino group is -NHMe or -NHEt, the arylamino group is benzylamino or halogenized benzylamino, and the alkylalkynyl group and the arylalkynyl group are phenylethynyl or benzylethynyl.

8. The pharmaceutical composition of claim 7, wherein Chemical Formula A-2 is the following Chemical Formula 2.

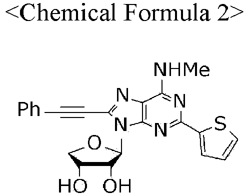

9. The pharmaceutical composition of claim 6, wherein the carbocyclic nucleoside derivative or salt has an IC₅₀ value of 7.5 µM or less, a CC₅₀ value of 50 or more and an SI value of 6.5 or more against SARS-CoV-2.
